# EUROPÄISCHE PATENTANMELDUNG

(11) **EP 3 311 848 A1**
(43) Veröffentlichungstag der Anmeldung: **25.04.2018**
(21) Anmeldenummer: 17191882.4
(22) Anmeldetag: 19.09.2017
(51) Int. Cl.: A61L 2/04, A61M 1/16

(54) **VERFAHREN ZUM DESINFIZIEREN EINES HYDRAULIKSYSTEMS EINER VORRICHTUNG ZUR EXTRAKORPORALEN BLUTBEHANDLUNG MITTELS HEISSDESINFEKTION SOWIE DERARTIGE VORRICHTUNG**

(30) Priorität: 19.09.2016 DE 102016117600
(71) Anmelder: B. Braun Avitum AG, 34212 Melsungen (DE)
(72) Erfinder: RITTER, Kai-Uwe, 91126 Rednitzhembach (DE)
(74) Vertreter: Winter, Brandl, Fürniss, Hübner, Röss, Kaiser, Polte - Partnerschaft mbB

(57) **Zusammenfassung**

Die Erfindung betrifft ein Verfahren zum Desinfizieren eines Hydrauliksystems (2) einer Vorrichtung (1) zur extrakorporalen Blutbehandlung mittels Heißdesinfektion, wobei in dem Hydrauliksystem (2) der Vorrichtung (1) enthaltenes Fluid auf eine für eine Heißdesinfektion ausreichende Spültemperatur unterhalb der Siedetemperatur erhitzt wird, und mit derart erhitztem Fluid zumindest ein Flussweg des Hydrauliksystems (2) über einen zum Erreichen eines vordefinierten A0-Werts ausreichenden Zeitraum gespült wird, wobei ein Maximaldruck oder eine Maximaltemperatur für einen Heißdesinfektionszyklus bestimmt wird, die dem Maximaldruck entsprechende Siedetemperatur bzw. der der Maximaltemperatur entsprechende Siededruck ermittelt wird und die Spültemperatur bzw. der Spüldruck anhand einer Korrelationstabelle bestimmt wird, die Korrelationen von A0-Werten mit Spülzeiträumen und Spültemperaturen enthält, derart, dass für einen angestrebten A0-Wert eine möglichst kurze Spüldauer gewählt wird. Die Erfindung betrifft des Weiteren eine Vorrichtung (1) zur extrakorporalen Blutbehandlung mit einem Hydrauliksystem (2) und einem mit dem Hydrauliksystem (2) strömungstechnisch verbundenen Vorlaufbehälter (4) für Hydraulikfluid, wobei zwischen dem Hydrauliksystem (2) und dem Vorlaufbehälter (4) eine Freifallstrecke zur Entgasung von Hydraulikfluid ausgebildet ist und der Vorlaufbehälter (4) über eine Druckausgleichleitung mit der Umgebung in Verbindung steht, wobei die Vorrichtung (1) einen Drucksensor (8) zur Bestimmung des Umgebungsdrucks und/oder einen Drucksensor (11) zur Bestimmung des Drucks im Vorlaufbehälter (4) aufweist.

## Beschreibung

Die Erfindung betrifft ein Verfahren zum Desinfizieren eines Hydrauliksystems einer Vorrichtung zur extrakorporalen Blutbehandlung mittels Heißdesinfektion, wobei in dem Hydrauliksystem der Vorrichtung enthaltenes Fluid auf eine für eine Heißdesinfektion ausreichende Spültemperatur unterhalb der Siedetemperatur erhitzt wird, und mit derart erhitztem Fluid zumindest ein Flussweg des Hydrauliksystems über einen zum Erreichen eines vordefinierten A0-Werts ausreichenden Zeitraum gespült wird. Sie betrifft des Weiteren eine Vorrichtung zur extrakorporalen Blutbehandlung mit einem Hydrauliksystem und einem mit dem Hydrauliksystem strömungstechnisch verbundenen Vorlaufbehälter für Hydraulikfluid, insbesondere eine solche Vorrichtung, die zur Durchführung des erfindungsgemäßen Verfahrens eingerichtet und geeignet ist.

In der chronischen Dialyse werden Vorrichtungen zur extrakorporalen Blutbehandlung in der Regel von mehreren Patienten nacheinander genutzt. Die Möglichkeit einer Übertragung von Keimen von einem Patienten zu einem anderen Patienten muss dabei unbedingt vermieden werden. Es ist Stand der Technik, dazu zwischen zwei Behandlungen eine Heißdesinfektion des Hydrauliksystems der Vorrichtung durchzuführen. Die dafür erforderliche Zeitdauer beträgt in der Regel je nach Vorrichtung und Hersteller zwischen 25 und 40 Minuten und setzt sich aus einer Aufheizphase (ca. 15 Minuten), einer Desinfektionsphase (ca. 15 Minuten) und einer Ausspül- und Abkühlphase (ca. 10 Minuten) zusammen. Es ist von Nachteil, dass die Vorrichtung während der Heißdesinfektion nicht für eine Blutbehandlung genutzt werden kann. Allenfalls kann die Vorrichtung über diesen Zeitraum mit einem neuen Blutschlauchsystem für einen nächsten Patienten bestückt werden. Die Zeitdauer der Desinfektion ist daher ein wesentlicher Faktor für einen wirtschaftlichen Einsatz einer Vorrichtung zur extrakorporalen Blutbehandlung und eine möglichst schnelle Desinfektion ist anzustreben.

In einem Hydrauliksystem einer Dialysemaschine gibt es konstruktionsbedingt unterschiedliche Abschnitte, in denen in Abhängigkeit von der dort bei einer Heißdesinfektion erreichten Temperatur jeweils ein spezifischer A0-Wert vorliegt. Abschnitte zwischen einem Vorlaufbehälter und dem Dialysator lassen sich relativ einfach erwärmen, so dass dort nach relativ kurzen Zeitspannen ausreichend hohe A0-Werte erreicht werden können. So kann je nach Gerätetyp in einem Bereich von dem Dialysator zum Beispiel innerhalb von ca. 15 bis 20 Minuten bei einer Temperatur von 82°C bis 86°C ein A0-Wert von etwa 1.400 bis ca. 4.700 erreichen. Hinter dem Dialysator können innerhalb von etwa 15 bis 20 Minuten nur Temperaturen von 80°C bis 84°C erreicht werden, was einen A0-Wert zwischen 900 und 3.000 zur Folge hat. Der aus mikrobiologischer Sicht kritische Bereich eines Hydrauliksystems einer Dialysemaschine befindet sich demnach hinter dem Dialysator. Um wirksam gegen zum Beispiel Hepatitis B zu sein, muss ein A0-Wert von wenigstens 3.000 sichergestellt sein.

Ein Problem bei zahlreichen bekannten Vorrichtungen zur extrakorporalen Blutbehandlung ist, dass zum Entgasen von Hydraulikfluid (Dialysierflüssigkeit) deren Hydrauliksystem zur Umgebung (Atmosphäre) hin offen ist. Die Entgasung erfolgt in der Regel über eine Freifallstrecke in einem Vorlaufbehälter. Sie erfolgt über einen Unterdruck, so dass die dabei entstehenden Gasblasen austreten und Gas in die Atmosphäre / Umgebung entweichen kann. Da das Hydrauliksystem offen ist, kann das zur Heißdesinfektion zu erhitzende Hydraulikfluid (Wasser) nicht über den jeweils vorliegenden Siedepunkt hinaus erhitzt werden. Da Vorrichtungen zur extrakorporalen Blutbehandlung universell einsetzbar sind und sein sollen, sie also auch in hoch liegenden Gegenden zum Beispiel bis hin zu 4000 Meter Meereshöhe, einsetzbar sein sollen, ist die Desinfektionstemperatur bei herkömmlichen Verfahren und Vorrichtungen auf einen Wert begrenzt, so dass das Hydraulikfluid auch in derart großer Höhe nicht siedet. Üblicherweise ist die maximale Desinfektionstemperatur von Vorrichtungen und Verfahren nach dem Stand der Technik daher auf etwa 85°C beschränkt. Bei einer solchen Temperatur ist zum Erreichen eines A0-Werts von 3.000 eine Expositionsdauer von etwa 15 Minuten erforderlich. Eine Übersicht über Expositionszeitdauern bei unterschiedlichen Temperaturen gibt die nachfolgende Tabelle:

| | 70°C | 75°C | 80°C | 85°C | 90°C |
|---|---|---|---|---|---|
| A0 = 600 | 100 Minuten | 31,5 Minuten | 10 Minuten | 3,16 Minuten | 1 Minute |
| A0 = 3.000 | 500 Minuten | 158 Minuten | 50 Minuten | 15,8 Minuten | 5 Minuten |

Ausgehend von dem vorstehend beschriebenen Stand der Technik liegt der vorliegenden Erfindung die Aufgabe zugrunde, die zuvor angeführten Nachteile zu beseitigen, insbesondere ein Verfahren zum Desinfizieren eines Hydrauliksystems einer Vorrichtung zur extrakorporalen Blutbehandlung mittels Heißdesinfektion sowie eine zur Durchführung eines solchen Verfahrens geeignete und eingerichtete Vorrichtung zu schaffen, wobei gegenüber dem Stand der Technik bei gleichem A0-Wert kürzere Desinfektionszeitdauern möglich sind.

Nach der Erfindung wird diese Aufgabe gelöst durch ein Verfahren zum Desinfizieren eines Hydrauliksystems einer Vorrichtung zur extrakorporalen Blutbehandlung mittels Heißdesinfektion, wobei
- in dem Hydrauliksystem der Vorrichtung enthaltenes Fluid, insbesondere Wasser oder Dialysierflüssigkeit, auf eine für eine Heißdesinfektion ausreichende Spültemperatur unterhalb der Siedetemperatur erhitzt wird, und
- mit derart erhitztem Fluid zumindest ein Flussweg des Hydrauliksystems über einen zum Erreichen eines vordefinierten A0-Werts ausreichenden Zeitraum gespült wird,
   dadurch gekennzeichnet, dass
- ein Maximaldruck oder eine Maximaltemperatur für einen Heißdesinfektionszyklus bestimmt wird,
- (in dem Fall, dass ein Maximaldruck bestimmt wurde,) die dem Maximaldruck entsprechende Siedetemperatur bzw. (in dem Fall, dass eine Maximaltemperatur bestimmt wurde,) der der Maximaltemperatur entsprechende Siededruck ermittelt wird und
- die Spültemperatur bzw. (und/oder) der Spüldruck anhand einer Korrelationstabelle bestimmt wird, die Korrelationen von A0-Werten mit Spülzeiträumen und Spültemperaturen enthält, derart, dass für einen angestrebten A0-Wert eine möglichst kurze Spüldauer gewählt wird.

Im Hinblick auf die Vorrichtung wird die Aufgabe gelöst durch eine Vorrichtung zur extrakorporalen Blutbehandlung mit einem Hydrauliksystem und einem mit dem Hydrauliksystem strömungstechnisch verbundenen Vorlaufbehälter für Hydraulikfluid (Dialysierflüssigkeit, Wasser), wobei zwischen dem Hydrauliksystem und dem Vorlaufbehälter eine Freifallstrecke zur Entgasung von Hydraulikfluid ausgebildet ist und der Vorlaufbehälter über eine Druckausgleichleitung mit der Umgebung in Verbindung steht, wobei die Vorrichtung einen Drucksensor zur Bestimmung des Umgebungsdrucks und/oder einen Drucksensor zur Bestimmung des Drucks im Vorlaufbehälter aufweist.

Insbesondere weist die Vorrichtung auf:
- eine Erhitzungseinrichtung zum Erhitzen des in dem Hydrauliksystem der Vorrichtung enthaltenen Fluids auf eine für eine Heißdesinfektion ausreichende Spültemperatur unterhalb der Siedetemperatur,
- eine Spüleinrichtung zum Spülen zumindest eines Flusswegs des Hydrauliksystems mit dem erhitztem Fluid über einen zum Erreichen eines vordefinierten A0-Werts ausreichenden Zeitraum,
- ein Bestimmungsmittel zum Bestimmen eines Maximaldrucks oder einer Maximaltemperatur für einen Heißdesinfektionszyklus,
- ein Ermittlungsmittel zum Ermitteln (, in dem Fall, dass ein Maximaldruck bestimmt wurde,) der dem Maximaldruck entsprechenden Siedetemperatur bzw. (, in dem Fall, dass eine Maximaltemperatur bestimmt wurde,) des der Maximaltemperatur entsprechenden Siededrucks und
- ein Bestimmungsmittel zum Bestimmen der Spültemperatur bzw. (und/oder) des Spüldrucks anhand einer Korrelationstabelle, die Korrelationen von A0-Werten mit Spülzeiträumen und Spültemperaturen enthält, derart, dass für einen angestrebten A0-Wert eine möglichst kurze Spüldauer gewählt wird.

Erfindungsgemäß können bevorzugt alle Verfahrensschritte automatisch ablaufen. Unter einem Hydrauliksystem einer Vorrichtung zur extrakorporalen Blutbehandlung im Sinne der Erfindung ist insbesondere ein Dialysierflüssigkeitsleitungssystem, ein Dialysierflüssigkeitskreislauf oder ein Hydraulikkreislauf einer solchen Vorrichtung zu verstehen. Der vordefinierte A0-Wert ist ein für eine Heißdesinfektion ausreichender Wert, zum Beispiel insbesondere ein A0-Wert von mindestens 3.000 zum Erreichen einer Wirksamkeit gegen Hepatitis B.

Es ist ein besonderer Vorteil der Erfindung, dass Heißdesinfektionen unabhängig von den jeweils vorliegenden Umgebungsbedingungen erfolgen können, die bisher stets einen beschränkenden Faktor dargestellt haben. Der eine Heißdesinfektion nach der Erfindung beschränkende Faktor ist nunmehr der erfindungsgemäß bestimmte Maximaldruck bzw. die Maximaltemperatur. Diese können entsprechend den jeweils vorliegenden Bedingungen, insbesondere entsprechend den jeweils vorliegenden Umgebungsbedingungen, bestimmt werden. Durch diese einsatzgemäße Bestimmung von Maximaldruck bzw. Maximaltemperatur, variabel und abgestimmt zum Beispiel auf die jeweils vorliegenden Umgebungsbedingungen, insbesondere auf die vorliegende Meereshöhe oder den Luftdruck je nach Wetterlage, kann eine Heißdesinfektion schnellstmöglich durchgeführt werden. Dies verringert "Stillstandzeiten" zwischen einzelnen Behandlungen und ermöglicht schnellere Behandlungen von mehr Patienten. Die Desinfektionszeit kann nach der Erfindung mit besonderem Vorteil automatisch an den sich aus der jeweilige Höhe ergebenden Luftdruck angepasst werden, wodurch sich in normalen bis niedrigen Lagen deutliche Vorteile in Form verkürzter Desinfektionszeiten ergeben. In großen Höhen können längere Desinfektionszeiten erforderlich sein, jedoch nicht auf längere Zeiträume, als sie nach dem Stand der Technik erforderlich sind.

Beispielsweise kann nach der Erfindung abhängig von einer gewünschten Spültemperatur (z.B. 105°C) ein variabler Druck, der sich aus der gewünschten Temperatur ergibt, eingestellt werden. Der Druck kann insbesondere derart bestimmt werden, dass der Siedepunkt über der gewünschten variablen Temperatur liegt, beispielsweise um ca. 5°C höher. Die für die Desinfektion erforderliche Zeitdauer ergibt sich durch eine Berechnung aus der gewünschten Temperatur und dem gewünschten A0-Wert. Andererseits kann nach der Erfindung zum Beispiel abhängig von einem gewünschten maximalen Druck, z.B. 1,0 bar, eine variable Spültemperatur, die sich aus dem gewünschten Druck ergibt, eingestellt werden. Die Temperatur wird so eingestellt, dass sie unter der sich für den gewünschten Druck ergebenden Siedepunkt liegt, z.B. 5°C darunter. Die für eine Desinfektion erforderliche Zeitdauer ergibt sich durch Berechnung aus der eingestellten Temperatur und dem gewünschten A0-Wert.

Vorteilhafte Ausführungsformen der Erfindung sind in den Unteransprüchen beansprucht und werden nachfolgend näher erläutert.

Nach einer Ausführungsform des Verfahrens nach der Erfindung ist der Maximaldruck der während des Desinfizierens herrschende Umgebungsdruck, insbesondere Luftdruck. In diesem Fall ist es besonders vorteilhaft, wenn die Vorrichtung einen Drucksensor zur Bestimmung des Umgebungsdrucks aufweist. Ein weiterer Vorteil ist, dass das Verfahren auf herkömmlichen Vorrichtungen zur extrakorporalen Blutbehandlung durchgeführt werden kann, bzw. auf solchen Vorrichtungen, bei denen das Hydrauliksystem zur Umgebung offen ist. Die zur Desinfektion verwendete Spültemperatur wird bei einer Ausführungsform des Verfahrens vorzugsweise derart bestimmt, dass sie knapp unter dem dem jeweils herrschenden Luftdruck entsprechenden Siedepunkt liegt. Auf eine Meereshöhe von zum Beispiel 300m über NN liegt der Siedepunkt von Wasser bei ca. 99°C. Nach dem erfindungsgemäßen Verfahren kann in einem solche Fall problemlos mit zum Beispiel 90°C oder 95°C (statt wie bislang nach dem Stand der Technik mit maximal 85°C) desinfiziert werden, wodurch sich zum Erreichen eines gewünschten A0-Werts bereits eine Verkürzung der für eine Heißdesinfektion erforderlichen Zeit um wenigstens fünf Minuten ergibt. Im Fall eines Einsatzes in großer Meereshöhe oder bei Tiefdruckwetter kann die Desinfektionstemperatur in entsprechender Weise abgesenkt und im Gegenzug die Zeitdauer für einen Desinfektionsvorgang entsprechend verlängert werden, um den gewünschten A0-Wert zu erreichen. Eine Desinfektion kann zum Beispiel bei nur 80°C über einen Zeitraum von ca. 50 Minuten erfolgen, um einen A0-Wert von 3.000 sicherzustellen.

Eine besonders ausgeprägte Verkürzung der zum Erreichen eines bestimmten A0-Werts erforderlichen Desinfektionszeit kann erzielt werden, indem die Spültemperatur knapp, insbesondere möglichst knapp, unter der dem Maximaldruck entsprechenden Siedetemperatur liegt. Zum Beispiel kann nach einer Ausführungsform des Verfahrens nach der Erfindung die anhand der Korrelationstabelle bestimmte Spültemperatur in einem Bereich von ca. 10°C bis ca. 0,5°C, vorzugsweise von ca. 7°C bis ca. 1°C, bevorzugter von ca. 5°C bis ca. 2°C, noch bevorzugter von ca. 4°C bis ca. 3°C unterhalb der dem Maximaldruck entsprechenden Siedetemperatur liegt. Sie kann innerhalb dieser Temperaturbereiche jeden beliebigen Temperaturwert annehmen.

Eine Ausführungsform der Erfindung erfordert, dass das Hydrauliksystem der Vorrichtung gegenüber der Umgebung abschließbar und abdichtbar ist und im Verlauf des Verfahrens abgeschlossen und abgedichtet wird. Dies kann insbesondere bewirkt werden, indem die Vorrichtung ein Ventil aufweist, mit dem ein Vorlaufbehälter einer Vorrichtung zur extrakorporalen Blutbehandlung gegenüber der Umgebung (Atmosphäre) absperrbar ist. Insbesondere kann der Vorlaufbehälter ein Ventil aufweisen, das es ermöglicht, ihn zur Atmosphäre hin entweder offen oder geschlossen zu betreiben.

Das Ventil kann nach einer Ausführungsform zum Desinfizieren der Hydraulikstrecke geschlossen werden. Die Desinfektion kann in besonders vorteilhafter Weise in einem geschlossenen System und vollkommen unabhängig vom jeweils vorliegenden Umgebungsluftdruck erfolgen. Nach erfolgter Desinfektion kann das Ventil für eine extrakorporale Blutbehandlung geöffnet werden, so dass weiterhin eine Entgasung unter Unterdruck in die Atmosphäre möglich ist. Man kann also auch sagen, dass das Ventil im Normalbetrieb offen ist, um ein Entlüften der Luft aus der Entgasung zu ermöglichen. Ein besonderer Vorteil dieser Ausführungsform ist, dass der zum Desinfizieren vorliegende Druck deutlich erhöht werden kann. Theoretisch könnte damit autoklaviert werden, also eine Desinfektionstemperatur von mehr als 120°C erzielt und eingestellt werden, wobei allerdings Grenzen gesetzt sind, da alle durch den Desinfketionsdruck belasteten Komponenten des Hydrauliksystems für die entsprechend höheren Temperaturen und Drücke (zum Beispiel 2bar) ausgelegt werden müssten und damit teurer werden. Es könnten allerdings A0-Werte im Millionenbereich erreicht werden.

Die vorstehende Ausführungsform kann besonders einfach bewirkt werden, indem der im Vorlaufbehälter vorliegende Druck mittels eines Drucksensors erfasst wird, der Teil der erfindungsgemäßen Vorrichtung sein kann. Der Druck kann mit dem Sensor insbesondere bei geschlossenem Hydrauliksystem fortlaufend kontinuierlich oder in Intervallen erfasst werden. Durch Erhitzen von Hydraulikmedium, zum Beispiel Wasser, steigt der Druck im Vorlaufbehälter an. Der in dem Vorlaufbehälter vorliegende Druck wird auf den bestimmten Maximaldruck geregelt. Zum Beispiel kann nach einer Ausführungsform der Erfindung der im Vorlaufbehälter vorliegende Druck mittels eines Überdruckventils geregelt werden. Insbesondere kann der Vorlaufbehälter ein Überdruckventil aufweisen, das verhindert, dass sich im Fehlerfall ein zu hoher Druck aufbauen kann. Das Überdruckventil kann den Vorlaufbehälter insbesondere gegenüber der Umgebung oder in die Umgebung entlüften und kann insbesondere zusätzlich zu einem das Hydrauliksystem schließenden Ventil vorliegen. Im Rahmen der vorstehenden Ausführungsform kann der Innendruck im Vorlaufbehälter zum Beispiel auf einen Wert von ca. 1000 mbar geregelt werden. Die Spültemperatur kann damit auf eine höhere vorteilhafte Temperatur gebracht bzw. geregelt werden, beispielsweise 90°C oder 95°C. Diese Temperatur liegt für die meisten Bauteile des Hydrauliksystems noch im Normalbereich, so dass das Verfahren auch mit herkömmlichen Vorrichtungen durchgeführt werden kann.

Der bestimmte Maximaldruck kann insbesondere auf Werten zwischen ca. 400 mbar und ca. 1500 mbar, vorzugsweise zwischen ca. 550 mbar und ca. 1200 mbar und besonders bevorzugt zwischen ca. 650 mbar und ca. 1100 mbar liegen.

Nach einer Ausführungsform der Vorrichtung der Erfindung weist diese eine Steuereinrichtung auf. Diese ist insbesondere eingerichtet, bestimmt und ausgebildet, alle oder einige der Verfahrensschritte des erfindungsgemäßen Verfahrens zu steuern oder auszuführen, insbesondere derart, dass diese automatisch ablaufen. Die Steuereinrichtung kann insbesondere bewirken, dass in dem Hydrauliksystem der Vorrichtung enthaltenes Fluid auf eine für eine Heißdesinfektion ausreichende Spültemperatur unterhalb der Siedetemperatur erhitzt wird, und mit derart erhitztem Fluid zumindest ein Flussweg des Hydrauliksystems über einen zum Erreichen eines vordefinierten A0-Werts ausreichenden Zeitraum gespült wird, ein Maximaldruck oder eine Maximaltemperatur für einen Heißdesinfektionszyklus bestimmt wird, die dem Maximaldruck entsprechende Siedetemperatur bzw. der der Maximaltemperatur entsprechende Siededruck ermittelt wird und die Spültemperatur bzw. der Spüldruck anhand einer Korrelationstabelle bestimmt wird, die Korrelationen von A0-Werten mit Spülzeiträumen und Spültemperaturen enthält, derart, dass für einen angestrebten A0-Wert eine möglichst kurze Spüldauer gewählt wird. Sie kann außerdem derart konfiguriert sein, dass der Maximaldruck der während des Desinfizierens herrschende Umgebungsdruck ist und/oder dass die Spültemperatur knapp unter der dem Maximaldruck entsprechenden Siedetemperatur liegt. Sie kann insbesondere derart konfiguriert sein, dass die anhand der Korrelationstabelle bestimmte Spültemperatur in einem Bereich von ca. 10°C bis ca. 0,5°C, vorzugsweise von ca. 7°C bis ca. 1°C, bevorzugter von ca. 5°C bis ca. 2°C, noch bevorzugter von ca. 4°C bis ca. 3°C unterhalb der dem Maimaldruck entsprechenden Siedetemperatur liegt.

Nach einer Ausführungsform kann das Hydrauliksystem mittels der Steuereinrichtung derart gesteuert werden, dass es gegenüber der Umgebung abgeschlossen und abgedichtet werden kann. Dies kann insbesondere erfolgen, indem das Ventil, mit dem der Vorlaufbehälter gegenüber der Umgebung absperrbar ist, durch die Steuereinrichtung steuerbar ist und gesteuert wird (die Steuereinrichtung also zum Steuern des Ventils eingerichtet ist). Dabei kann das Ventil zum Desinfizieren der Hydraulikstrecke mittels der Steuereinrichtung geschlossen und nach erfolgter Desinfektion für eine extrakorporale Blutbehandlung geöffnet werden.

Außerdem kann die Steuereinrichtung eingerichtet sein, um den Drucksensor zur Erfassung des im Vorlaufbehälter vorliegenden Drucks zu steuern, insbesondere derart, dass bei geschlossenem Hydrauliksystem fortlaufend kontinuierlich oder in Intervallen erfasst wird, und der in dem Vorlaufbehälter vorliegende Druck auf den bestimmten Maximaldruck geregelt wird. Insbesondere kann mittels der Steuereinrichtung der im Vorlaufbehälter vorliegende Druck mittels des Überdruckventils geregelt werden, das den Vorlaufbehälter gegenüber der Umgebung entlüften kann. Die Steuereinrichtung kann derart konfiguriert sein, dass der bestimmte Maximaldruck zwischen ca. 400 mbar und ca. 1500 mbar, vorzugsweise zwischen ca. 550 mbar und ca. 1200 mbar und besonders bevorzugt zwischen ca. 650 mbar und ca. 1100 mbar liegt.

Durch die Erfindung können insbesondere die folgenden Vorteile bzw. Verbesserungen bewirkt werden:
- Verkürzung der Desinfektionszeit für identische A0-Werte: Bereits bei einer Spültemperatur von 90 Grad ergibt sich für eine A0-Wert von 3.000 eine Desinfektionsdauer von nur 5 Minuten gegenüber einer Desinfektionsdauer von ca. 15 Minuten bei einer Temperatur von nur 85°C, wie üblich.
- Eine Spültemperatur von ca. 90°C oder 95°C ist eine günstige Auslegung, da die Spezifikation der meisten Bauteile dort noch in einem günstigen Bereich liegt.

Die Erfindung wird im Folgenden anhand von beispielhaften, nicht einschränkenden und in den angehängten Figuren gezeigten Ausführungsformen näher erläutert. Dabei zeigt:
Fig. 1 eine schematische Darstellung einer Vorrichtung zur extrakorporalen Blutbehandlung nach der Erfindung in einer ersten Ausführungsform,
Fig. 2 eine schematische Darstellung einer Vorrichtung zur extrakorporalen Blutbehandlung nach der Erfindung in einer zweiten Ausführungsform, und

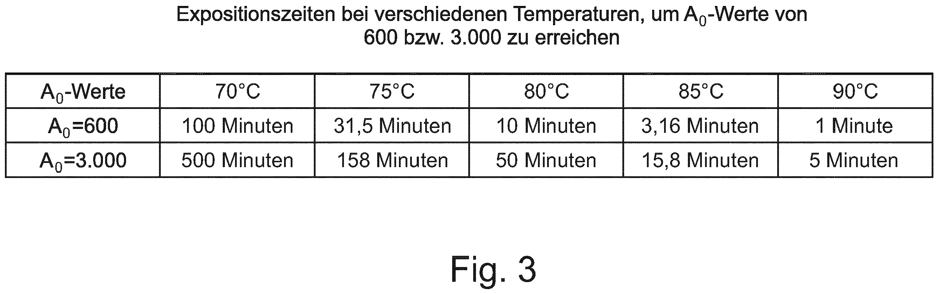

Fig. 3 eine Korrelationstabelle mit Zuordnungen von A0-Werten und Spültemperaturen.

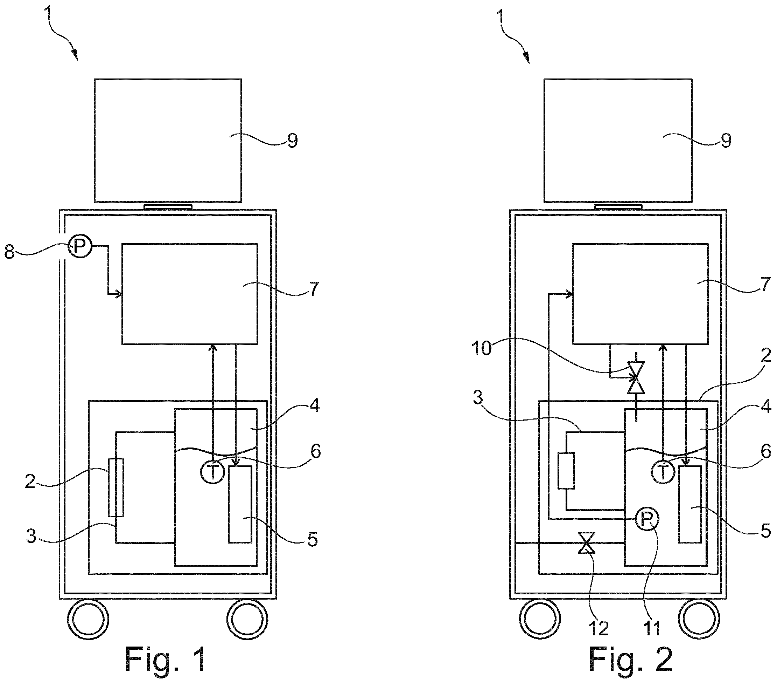

Die Figuren 1 und 2 zeigen zwei Ausführungsformen einer erfindungsgemäßen Vorrichtung 1, zum Beispiel ein Dialysegeräte 1. Gleiche Bestandteile werden gleich bezeichnet und beschrieben. Die Vorrichtung 1 weist ein Hydrauliksystem 2 auf, das eine Hydraulikstrecke 3 umfasst. Das Hydrauliksystem 2 kann ein Dialysierflüssigkeitsleitungssystem, einen Dialysierflüssigkeitskreislauf oder einen Hydraulikkreislauf ausbilden. Es weist einen Vorlaufbehälter 4 oder Vorratsbehälter 4 auf, in dem Hydraulikmedium, zum Beispiel Wasser, gespeichert ist und für eine Verwendung vorbereitet wird.

Zur Temperierung des Fluids weist die Vorrichtung 1 ein Heizelement 5 (Heizung) auf. Dieses ist in den gezeigten Ausführungsformen in dem Vorlaufbehälter 4 platziert. Die Temperatur des Fluids im Vorlaufbehälter 4, die der Spültemperatur bei einem Heißdesinfektionsvorgang entspricht, ist mit einem Temperatursensor 6 zu erfassen. Die Vorrichtung 1 weist außerdem eine Steuerung 7 auf. In dieser sind unter anderem Korrelationstabellen hinterlegt, in denen Zuordnungen zwischen einer Mehrzahl von A0-Werten und Spültemperaturen enthalten sind. Ein Beispiel einer solchen Korrelationstabelle zeigt Figur 4.

Die Vorrichtung der Figur 1 besitzt einen Drucksensor 8 oder Außendrucksensor 8, mit dem der Umgebungs- oder Atmosphärendruck gemessen und erfasst werden kann. Das Hydrauliksystem 2 der Vorrichtung 1 der Figur 1 ist ein offenes System, es unterliegt also dem die Vorrichtung 1 umgebenden Umgebungsdruck, der auch innerhalb des Hydrauliksystems herrscht. In die Steuerung 7 werden unter anderem als Eingangsgrößen die mit dem Drucksensor 8 ermittelte Umgebungstemperatur sowie die Ist-Temperatur des Fluids im Vorratsbehälter 4 eingespeist. Über einen Monitor 9 oder eine Bedieneinheit 9 kann ein Nutzer einen gewünschten A0-Wert eingeben. Anhand des Umgebungsdrucks ermittelt die Steuerung die diesem Druck entsprechende Siedetemperatur und steuert die Spültemperatur des Fluids im Vorlaufbehälter 4 auf einen Wert leicht unterhalb dieser Siedetemperatur. Auf diese Weise wird die Spültemperatur entsprechend dem gemessenen Außendruck so hoch wie möglich eingestellt, wobei jedoch ein Sieden vermieden wird und für den ausgewählten A0-Wert die kürzeste mögliche Desinfektionsdauer durchgeführt wird.

Das Hydrauliksystem 2 der Vorrichtung 1 der Figur 2 ist über ein Ventil 10 mit der Umgebung verbunden und kann dadurch während einer Blutbehandlung als offenes System und während einer Heißdesinfektion als geschlossenes System betrieben werden. Das Ventil 10 ist also während einer Blutbehandlung offen und während einer Heißdesinfektion geschlossen.

Ein Nutzer kann in die Steuerung 7 einen Maximaldruck oder eine maximale Spültemperatur eingeben. Anhand des gewünschten Maximalwerts ermittelt die Steuerung 7 die dem Maximaldruck entsprechende Siedetemperatur bzw. den der Maximaltemperatur entsprechenden Siededruck. Nachfolgend wird durch die Steuerung 7 die Spültemperatur bzw. der Spüldruck anhand der darin hinterlegten Korrelationstabelle bestimmt. Diese enthält Korrelationen von A0-Werten mit Spülzeiträumen und Spültemperaturen. Dies ermöglicht, dass für einen angestrebten A0-Wert eine möglichst kurze Spüldauer gewählt wird.

Ein Innendrucksensor 11, der den Druck innerhalb des Vorlaufbehälters 4 erfasst, ist in diesem angeordnet und mit der Steuerung datentechnisch verbunden. Bei Abweichungen von den jeweils gewünschten Sollwerten kann die Steuerung 7 die Heizleistung der Heizung 5 und/oder den Innendruck im Vorlaufbehälter 4 über das Ventil 10 oder zusätzliches Überdruckventil 12 regeln.

### Bezugszeichen

- 1: Vorrichtung zur extrakorporalen Blutbehandlung, Dialysevorrichtung
- 2: Hydrauliksystem, Dialysierflüssigkeitssystem
- 3: Hydraulikstrecke, Dialysierflüssigkeitsstrecke
- 4: Vorlaufbehälter
- 5: Heizelement
- 6: Temperatursensor
- 7: Steuerung
- 8: Drucksensor, Außendrucksensor
- 9: Monitor, Bedieneinheit
- 10: Ventil
- 11: Innendrucksensor
- 12: Überdruckventil

## Patentansprüche

1. Verfahren zum Desinfizieren eines Hydrauliksystems (2) einer Vorrichtung (1) zur extrakorporalen Blutbehandlung mittels Heißdesinfektion, wobei
- in dem Hydrauliksystem (2) der Vorrichtung (1) enthaltenes Fluid auf eine für eine Heißdesinfektion ausreichende Spültemperatur unterhalb der Siedetemperatur erhitzt wird, und
- mit derart erhitztem Fluid zumindest ein Flussweg des Hydrauliksystems (2) über einen zum Erreichen eines vordefinierten A0-Werts ausreichenden Zeitraum gespült wird,
**dadurch gekennzeichnet, dass**
- ein Maximaldruck oder eine Maximaltemperatur für einen Heißdesinfektionszyklus bestimmt wird,
- die dem Maximaldruck entsprechende Siedetemperatur bzw. der der Maximaltemperatur entsprechende Siededruck ermittelt wird und
- die Spültemperatur bzw. der Spüldruck anhand einer Korrelationstabelle oder einer Berechnung bestimmt wird, die Korrelationen von A0-Werten mit Spülzeiträumen und Spültemperaturen enthält, derart, dass für einen angestrebten A0-Wert eine möglichst kurze Spüldauer gewählt wird.

2. Verfahren nach Anspruch 1, **dadurch gekennzeichnet, dass** der Maximaldruck der während des Desinfizierens herrschende Umgebungsdruck ist.

3. Verfahren nach Anspruch 1 oder 2, **dadurch gekennzeichnet, dass** die Spültemperatur knapp unter der dem Maximaldruck entsprechenden Siedetemperatur liegt.

4. Verfahren nach einem der vorstehenden Ansprüche, **dadurch gekennzeichnet, dass** die anhand der Korrelationstabelle bestimmte Spültemperatur in einem Bereich von ca. 10°C bis ca. 0,5°C, vorzugsweise von ca. 7°C bis ca. 1°C, bevorzugter von ca. 5°C bis ca. 2°C, noch bevorzugter von ca. 4°C bis ca. 3°C unterhalb der dem Maximaldruck entsprechenden Siedetemperatur liegt.

5. Verfahren nach einem der vorstehenden Ansprüche, **dadurch gekennzeichnet, dass** der bestimmte Maximaldruck zwischen ca. 400 mbar und ca. 1500 mbar, vorzugsweise zwischen ca. 550 mbar und ca. 1200 mbar und besonders bevorzugt zwischen ca. 650 mbar und ca. 1100 mbar liegt.

6. Vorrichtung (1) zur extrakorporalen Blutbehandlung mit einem Hydrauliksystem (2) und einem mit dem Hydrauliksystem (2) strömungstechnisch verbundenen Vorlaufbehälter (4) für Hydraulikfluid, wobei zwischen dem Hydrauliksystem (2) und dem Vorlaufbehälter (4) eine Freifallstrecke zur Entgasung von Hydraulikfluid ausgebildet ist und der Vorlaufbehälter (4) über eine Druckausgleichleitung mit der Umgebung in Verbindung steht, **dadurch gekennzeichnet, dass** die Vorrichtung (1) einen Drucksensor (8) zur Bestimmung des Umgebungsdrucks und/oder einen Drucksensor (11) zur Bestimmung des Drucks im Vorlaufbehälter (4) und eine Steuereinrichtung aufweist, wobei die Steuereinrichtung dazu vorgesehen und angepasst ist, ein Verfahren gemäß einem der Ansprüche 1 bis 5 durchzuführen.

7. Vorrichtung (1) nach Anspruch 6, **dadurch gekennzeichnet, dass** die Druckausgleichleitung mittels eines Ventils abzusperren und zu öffnen ist.

8. Vorrichtung nach einem der Ansprüche 6 oder 7, **dadurch gekennzeichnet, dass** das Hydrauliksystem (2) zur Umgebung offen ist.

9. Vorrichtung nach einem der Ansprüche 6 bis 8, **dadurch gekennzeichnet, dass** das Hydrauliksystem (2) gegenüber der Umgebung abschließbar und abdichtbar ist.

10. Vorrichtung nach Anspruch 9, **dadurch gekennzeichnet, dass** diese ein Ventil (10) aufweist, mit dem ein Vorlaufbehälter (4) einer Vorrichtung (2) zur extrakorporalen Blutbehandlung gegenüber der Umgebung absperrbar ist, wobei das Ventil (10) zum Desinfizieren der Hydraulikstrecke (2) geschlossen wird und nach erfolgter Desinfektion für eine extrakorporale Blutbehandlung geöffnet wird.

11. Vorrichtung nach Anspruch 9 oder 10, **dadurch gekennzeichnet, dass** diese einen Drucksensor (11) aufweist, wobei der im Vorlaufbehälter (4) vorliegende Druck mittels des Drucksensors (11) erfasst wird, insbesondere bei geschlossenem Hydrauliksystem (2) fortlaufend kontinuierlich oder in Intervallen erfasst wird, und der in dem Vorlaufbehälter (4) vorliegende Druck auf den bestimmten Maximaldruck geregelt wird.

12. Vorrichtung nach Anspruch 11, **dadurch gekennzeichnet, dass** der im Vorlaufbehälter (4) vorliegende Druck mittels eines Überdruckventils geregelt wird, das den Vorlaufbehälter (4) gegenüber der Umgebung entlüften kann.
